(19) Europäisches Patentamt
European Patent Office
Office européen des brevets

(11) Veröffentlichungsnummer: **0 312 697 B1**

(12) # EUROPÄISCHE PATENTSCHRIFT

(45) Veröffentlichungstag der Patentschrift: **21.04.93**

(51) Int. Cl.$^5$: **A61K 31/225**

(21) Anmeldenummer: **88109783.6**

(22) Anmeldetag: **20.06.88**

Teilanmeldung 92114477.0 eingereicht am 20/06/88.

Die Akte enthält technische Angaben, die nach dem Eingang der Anmeldung eingereicht wurden und die nicht in dieser Patentschrift enthalten sind.

(54) **Pharmazeutische Zubereitungen zur Behandlung der Psoriasis, psoriatischen Arthritis, Neurodermitis und Enteritis regionalis Crohn.**

(30) Priorität: **19.10.87 US 109780**

(43) Veröffentlichungstag der Anmeldung: **26.04.89 Patentblatt 89/17**

(45) Bekanntmachung des Hinweises auf die Patenterteilung: **21.04.93 Patentblatt 93/16**

(84) Benannte Vertragsstaaten: **AT BE CH DE ES FR GB GR IT LI LU NL SE**

(56) Entgegenhaltungen:
**DE-A- 2 530 372**
**DE-A- 2 621 214**

**DIALOG 6398887, Embase (Exerpta Medica), no. 82-89115524; W. BAYARD et al.&NUM;**

**W. Schweckendiek, "FUMARSÄURE BEI PSORIASIS EINE NEUE ENTTÄUSCHUNG", 1960&NUM;**

(73) Patentinhaber: **Speiser, Peter Paul, Prof. Dr.**
**Freudenbergstrasse 101/D2**
**CH-8044 Zürich(CH)**

Patentinhaber: **Joshi, Rajendra K., Dr.**
**Badenerstrasse 795**
**CH-8044 Zürich(CH)**

(72) Erfinder: **Speiser, Peter Paul, Prof. Dr.**
**Freudenbergstrasse 101/D2**
**CH-8044 Zürich(CH)**
Erfinder: **Joshi, Rajendra K., Dr.**
**Badenerstrasse 795**
**CH-8044 Zürich(CH)**

(74) Vertreter: **Schwabe - Sandmair - Marx**
**Stuntzstrasse 16**
**W-8000 München 80 (DE)**

EP 0 312 697 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**ACTA MEDICA EMPIRICA, Bd. 30, Heft 8, Aug. 1981; Seiten 613-621&NUM;**

**DER HAUTARZT, Bd. 36, Springer-Verlag, 1985; G. ALBRECHT, Seiten 77-82&NUM;**

**PHARMAKOLOGIE UND TOXIKOLOGIE, 4. Aufl.; Seiten 317-318&NUM;**

## Beschreibung

Die Erfindung betrifft die Verwendung bestimmter Fumarsäuremonoalkylestersalze allein oder in Verbindung mit einem Dialkyfumarat zur Herstellung einer pharmazeutischen Zubereitung zur Behandlung der psoriatischen Arthritis, Neurodermitis und Enteritis regionalis Crohn. Die Erfindung betrifft ferner eine pharmazeutische Zubereitung zur Behandlung der Psoriasis, die eine oder mehrere Salze des Fumarsäuremonomethylesters gegebenenfalls zusammen mit Dialkylfumarat als Wirkstoff enthält. Diese Zubereitungen enthalten keine Fumarsäure per se.

Pharmazeutische Zubereitungen, die nach Verabreichung bei ihrem biologischen Abbau in den Zitronensäurezyklus einmünden oder diesem angehören, gewinnen meist in hoher Dosierung immer mehr an therapeutischem Wert, da man mit ihrer Hilfe kryptogenetisch bedingte Krankheiten zu lindern oder zu heilen vermag.

So hemmt Fumarsäure das Wachstum des Ehrlich-Ascites-Tumors bei Mäusen, vermindert die toxischen Effekte von Mitomycin C und Aflatoxin (K. Kuroda, M. Akao, Biochem. Pharmacol. 29, 2839-2844 (1980) / Gann. 72, 777-782 (1981) / Cancer Res. 36, 1900-1903 (1976)) und besitzt eine antipsoriatische sowie antimikrobielle Wirkung (C. N. Huhtsnen, J. Food Sci. 48, 1574 (1983) / M. N. Islam, U.S.-Patent 4 346 118 vom 24. August 1982 / C. A. 97, 161317b (1982)).

Hohe Verabreichungsdosen von Fumarsäure oder ihrer bisher bekannten Derivate wie Dihydroxyfumarsäure, Fumaramid und Fumaronitril besitzen bei parenteraler, dermaler, insbesondere aber peroraler Verabreichung eine derart unzumutbare Nebenwirkungsrate und hohe Toxizität (P. Holland, R. G. White, Brit. J. Dermatol. 85, 259-263 (1971) / M. Hagedorn, K. W. Kalkoff, G. Kiefer, D. Baron, J. Hug, J. Petres, Arch. Derm. Res. 254, 67-73 (1975)), daß bisher meist von einer solchen Therapie abgesehen werden mußte.

In der europäischen Patentanmeldung 18 87 49 sind bereits Fumarsäurederivate und sie enthaltende pharmazeutische Zubereitungen zur Behandlung der Psoriasis beschrieben.

Aus der DE-A 25 30 372 sind pharmazeutische Zubereitungen zur Behandlung der Psoriasis bekannt, die eine Mischung aus Fumarsäure und weitere Fumarsäurederivaten enthalten. Der Anteil der Fumarsäure ist obligatorisch.

Die DE-A-26 21 214 beschreibt Arzneimittel zur Behandlung der Psoriasis, die Fumarsäuremonoethylester und dessen Mineralsalze als Wirkstoff enthalten.

Aus der Publikation "Hautarzt (1987) 279-285" ist die Verwendung von Fumarsäuremonoethylestersalze (Ca, Zn, Mg) und Fumarsäuredimethylester für die Psoriasisbehandlung bekannt.

Es wurde nunmehr überraschend gefunden, daß eine insgesamt erheblich verbeserte Wirkung in der Behandlung der Psoriasis durch eine pharmazeutische Zubereitung erzielt werden kann, die eine oder mehrere Verbindungen aus der Gruppe der Calcium-, Magnesium-, Zink- und Eisensalze von Fumarsäuremonomethylester der allgemeinen Formel

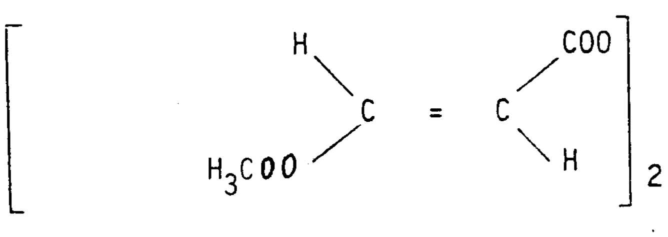
Ca(Mg, Zn, Fe),

allein oder vorzugsweise im Gemisch mit Dialkylfumarat der Formel

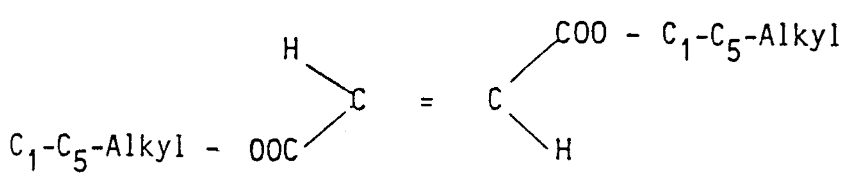

und üblichen pharmazeutisch verträglichen Hilfs- und Trägerstoffe enthält. Diese Zubereitungen enthalten keine Fumarsäure per se.

Es wurde eine ebenfalls verbesserte Wirkung bei der Behandlung der psoriatischen Arthritis, Neurodermitis und Enteritis regionalis Crohn durch pharmazeutische Zubereitungen gefunden, die die Verwendung einer oder mehrerer Verbindungen aus der Gruppe der Calcium-, Magnesium-, Zink- und Eisensalze von Fumarsäuremonoalkylester der allgemeinen Formel

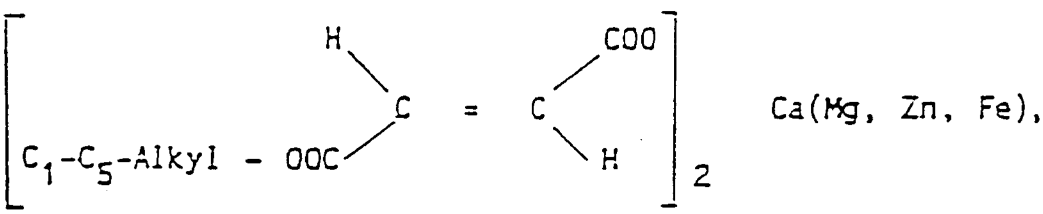

gegebenenfalls im Gemisch mit Dialkylfumarat der Formel

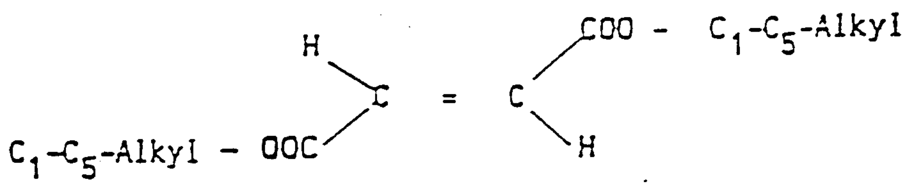

und übliche pharmazeutische Hilfs- und Trägerstoffe vorsehen.

Bevorzugte Zubereitungen gemäß der Erfindung enthalten das Calciumsalz des Fumarsäure-Monoethylesters, das Calciumsalz des Fumarsäure-Monoethylesters im Gemisch mit Dimethylfumarat, das Calcium- und Zinksalz des Fumarsäure-Monoethylesters im Gemisch mit Dimethylfumarat oder das Calcium-, Magnesium- und Zinksalz des Fumarsäure-Monoethylesters im Gemisch mit Dimethylfumarat. bzw. die jeweiligen Salze des Fumarsäuremonomethylesters.

Zur oralen Verabreichung sind besonders Zubereitungen geeignet, die das Calciumsalz des Fumarsäure-Monoalkylesters bzw. -monomethylesters in einer Menge von 100 bis 300 mg enthalten, wobei das Gesamtgewicht der Wirkstoffe 100 bis 300 mg beträgt.

Weitere bevorzugte orale Verabreichungsformen enthalten 10 bis 290 Gew.-Teile des Calciumsalzes des Fumarsäure-Monoalkylesters und 290 bis 10 Gew.-Teile Dimethylfumarat, 10 bis 250 Gew.-Teile des Calciumsalzes des Fumarsäure-Monoalkylesters, 1 bis 50 Gew.-Teile Dimethylfumarat und 1 bis 50 Gew.-Teile des Zinksalzes des Fumarsäure-Monoalkylesters oder 10 bis 250 Gew.-Teile des Calciumsalzes des Fumarsäure-Monoalkylesters, 250 bis 10 Gew.-Teile Dimethylfumarat, 1 bis 50 Gew.-Teile des Magnesiumsalzes des Fumarsäure-Monoalkylesters und 1 bis 50 Gew.-Teile des Zinksalzes des Fumarsäure-Monoalkylesters bzw. des Monomethylesters, wobei jeweils das Gesamtgewicht der Wirkstoffe 100 bis 300 mg beträgt.

Für den systemischen Einstieg in die Behandlung bzw. den Ausstieg ist eine niedrige Dosierung vorteilhaft, die beispielsweise aus 30,0 mg Dimethylfumarat, 67,0 mg des Calciumsalzes von Monoethylfumarat, 5,0 mg des Magnesiumsalzes von Monoethylfumarat und 3,0 mg des Zinksalzes von Monoethylfumarat bzw. von Monomethylfumarat enthält.

Für die therapeutische Dosierung nach einer Einstiegsphase kann beispielsweise eine Dosierung von 120,0 mg Dimethylfumarat, 87,0 mg des Calciumsalzes des Monoethylfumarats, 5,0 mg des Magnesiumsalzes des Monoethylfumarats und 3,0 mg des Zinksalzes des Monoethylfumarats bzw. des Monomethylfumarats zur Anwendung kommen.

Die in den erfindungsgemäßen Zubereitungen enthaltenen Fumarsäurederivate werden beispielsweise dadurch erhalten, daß man eine Verbindung der folgenden Formel

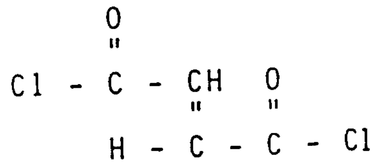

a) mit 2 Mol Alkylalkohol (ROH) zum Diester kondensiert und anschließend kontrolliert zum Monoester hydrolysiert, oder
b) mit 1 Mol eines entsprechenden Alkylalkohols (ROH) kondensiert und das erhaltene Monosäurechlorid zur Säure hydrolysiert, oder
c) Fumarsäure direkt mit 2 Mol Alkylalkohol (ROH) gemäß Anspruch 1 zu einem Diester kondensiert und anschließend kontrolliert zum Monoester hydrolysiert, oder
d) Maleinsäure oder Maleinsäureanhydrid direkt mit 1 - 2 Mol des entsprechenden Alkylalkohols (ROH) nach Anspruch 1 zu einem Mono- oder Diester kondensiert und anschließend katalytisch zum entsprechenden Fumarsäurederivat isomerisiert.

Die Salze der Fumarsäure-monoalkylester können dadurch erhalten werden, daß man eine Verbindung der allgemeinen Formel

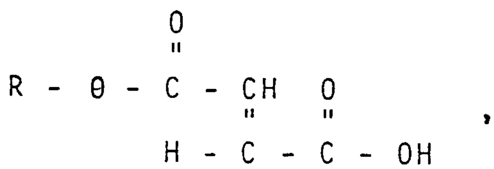

in der R eine $C_1$-$C_5$- Alkylgruppe bedeutet, mit einem halben Mol Ca-, Mg- oder Zn-Hydroxid oder -Oxid in Toluol zur Umsetzung bringt und das während der Reaktion gebildete Wasser azeotrop entfernt.

## Beispiel 1

### Herstellung von Filmtabletten mit magenresistentem Ueberzug enthaltend 210 mg Monoethylfumarat-Ca-Salz entsprechend 150 mg Fumarsäure:

21,000 kg Monoethylfumarat-Calciumsalz werden zerkleinert, gemischt und mittels eines Siebes 800 unter entsprechenden Vorsichtsmassnahmen (Atemmaske, Handschuhe, Schutzanzug etc.) homogenisiert. Anschliessend wird ein Hilfsstoffgemisch folgender Zusammensetzung hergestellt: 20,000 kg Stärkederivat (STA-RX 1500 ®), 2,000 kg mikrokristalline Cellulose (Avicel PH 101 ®), 0,600 kg Polyvinylpyrolidone (PVP, Kollidon ® 25), 4,000 kg Primogel ®, 0,300 kg kolloidaler Kieselsäure (Aerosil ®). Das gesamte Pulvergemisch wird mit dem Wirkstoff versetzt und mittels eines Siebes 200 homogenisiert und mit einer 2%igen wässrigen Lösung von Polyvinylpyrolidon (Kollidon ® K30) auf übliche Weise zu einem Bindemittelgranulat verarbeitet und in trockenem Zustand mit der äusseren Phase gemischt. Diese besteht aus 2,000 kg eines sogenannten FST-Komplexes, enthaltend 80 % Talk, 10% Kieselsäure und 10 % Magnesiumstearat. Es wird anschliessend auf übliche Weise zu gewölbten Tabletten von 500 mg Gewicht und 11,5 mm Durchmesser gepresst. Anstelle dieser klassischen Tablettiermethoden können auch andere Methoden zur Herstellung von Tabletten angewendet werden, die Direkttablettierung sowie feste Dispersionen nach der Schmelzmethode und der Sprühtrocknungsmethode.

### Magenresistenz:

Es wird eine Lösung von 2,250 kg Hydroxypropylmethylcellulosephthalat (HPMCP, Pharmacoat HP 50 ®) in einem Lösungsmittelgemisch von 2,50 l demineralisiertem Wasser, 13,00 l Aceton Ph.Helv.VII und 13,00 l Ethanol 94 Gew.-% gelöst und die Lösung mit 0,240 kg Rizinusöl (Ph. Eur. II) versetzt. Die Lösung wird im Dragierkessel auf traditionelle Weise in Portionen auf die Tablettenkerne aufgeleert oder aufgesprüht bzw. in einem Wirbelschichtapparat entsprechender Konstruktion aufgetragen.

Nach entsprechender Trocknung wird anschliessend der Filmüberzug angebracht. Dieser setzt sich zusammen aus einer Lösung von Eudragit ® E 12,5% 4,800 kg. Farblack ZLT 2 blau (Siegle) 0,210 kg,

Titan (VI)-oxyd Kronos RN 56 0,520 kg. Talk (Ph. Eur.) 0,340 kg und Polyethylenglycol 6000 Ph. Helv. VII 0,120 kg. in einem Lösungsgemisch von 8,200 kg 2-Propanol, Ph. Helv. VII, 0,060 kg Glycerintriacetat und 0,200 kg Aqua demineralisata. Nach homogener Verteilung im Dragierkessel oder Wirbelschichtbett wird getrocknet und auf übliche Weise poliert.

Beispiel 2

Herstellung von magenresistenten Kapseln enthaltend 86,5 mg Monoethylfumarat Ca-Salz und 110,0 mg Dimethylfumarat
entsprechend insgesamt 150 mg Fumarsäure

8,650 kg Monoethylfumarat Ca-Salz und 11,000 kg Dimethylfumarat werden mit einen Gemisch bestehend aus 15,000 kg Stärke, 6,000 kg Lactose Ph. Helv. VII, 2,000 kg mikrokristalliner Cellulose (Avicel ®), 1,000 kg Polyvinylpyrolidon (Kollidon ® 25) und 4,000 kg Primogel ® intensiv gemischt und mittels eines Siebes 800, unter Beachtung entsprechender Schutzmassnahmen (Atemmaske, Handschuhe, Schutzanzug etc.), homogenisiert. Das gesamte Pulvergemisch wird mit eine 2%igen wässrigen Lösung von Polyvinylpyrolidon (Kollidon ® 25) auf übliche Weise zu einem Bindemittelgranulat verarbeitet und in getrocknetem Zustand mit der äusseren Phase gemischt. Diese besteht aus 0,350 kg kolloidaler Kieselsäure (Aerosil ®), 0,500 kg Mg-Stearat und 1,500 kg Talkum Ph. Helv. VII. Das homogene Gemisch wird anschliessend in entsprechende Kapseln in Portionen von 500,0 mg abgefüllt, welche abschliessend auf übliche Weise mit einem magenresistenten Ueberzug, bestehend aus Hydroxypropylmethylcellulosestearat und Rizinusöl als Weichmacher, versehen werden. Die Abfüllung kann ebenfalls anstelle von Hartgelatinekapseln in entsprechende magenresistente Kapseln, bestehend aus einem Gemisch von Celluloseacetatphthalat (CAP) und Hydroxypropylethylcellulosephthalat (HPMCP) erfolgen.

Beispiel 3

Herstellung von magenresistenten Kapseln, enthaltend 203,0 mg Monoethylfumarat Ca-Salz sowie 5,0 mg Monoethylfumarat Mg-Salz und 3,0 mg Monoethylfumarat Zn-Salz
entsprechend insgesamt 150 mg Fumarsäure

20,300 kg Monoethylfumarat Ca-Salz sowie 0,500 kg Monoethylfumarat Mg-Salz und 0,300 kg Monoethylfumarat Zn-Salz werden zerkleinert, intensiv gemischt und unter entsprechenden Schutzmassnahmen (Atemmaske, Handschuhe, Schutzanzug etc.) mittels eines Siebes 800 homogenisiert. Diesem Wirkstoffgemisch wird ein homogenes Pulvergemisch folgender Zusammensetzung untergemischt: sprühgetrocknete Lactose 12,900 kg, kolloidale Kieselsäure 1,000 kg, mikrokristalline Cellulose (Avicel ®) 2,000 kg, Magnesiumstearat (Ph. Helv. VII) 1,000 kg und Talk (Ph. Helv. VII) 2,000 kg. Das gesamte Pulvergemisch wird nochmals mittels eines Siebes 200 homogenisiert und anschliessend in Hartgelatine-Steckkapseln zu 400 mg Nettogewicht eingefüllt und verschlossen. Das Ueberziehen mit einem magenresistenen Ueberzug erfolgt wie in Beispiel 2.

Beispiel 4

Herstellung von magenresistenten Tabletten, enthaltend 87,0 mg Monoethylfumarat Ca-Salz, 120,0 mg Dimethylfumarat, 5,0 mg Monoethylfumarat Mg-Salz und 3,0 mg Monoethylfumarat Zn-Salz
ensprechend 164 mg Fumarsäure ("Forte"-Tabletten)

12,000 kg Fumarsäuredimethylester, 8,700 kg Monoethylfumarat Ca-Salz, 0,500 kg Monoethylfumarat Mg-Salz und 0,300 kg Monoethylfumarat Zn-Salz werden zerkleinert, intensiv gemischt und mittels eines Siebes 800 homogenisiert, unter Beachtung entsprechender Schutzmassnahmen (Atemmaske, Handschuhe, Schutzanzug etc.). Es wird ein Hilfsstoffgemisch folgender Zusammensetzung, auf ähnliche Weise wie unter Beispiel 1 aufgeführt, hergestellt: Stärkederivat (STA-RX 1500 ®) 18,000 kg, mikrokristalline Cellulose (Avicel pH 101 ®) 0,300 kg, Polyvinylpyrolidone (PVP, Kollidon ® 120) 0,750 kg, Primogel ® 4,000 kg und kolloidale Kieselsäure (Aerosil ®) 0,250 kg.

Hilfsstoffe und Wirkstoffgemisch werden intensiv gemischt und mittels eines Siebes 200 homogenisiert. Das Ganze wird mit einer 2%igen wässrigen Lösung von Polyvinylpyrolidon (Kollidon ® K25) auf übliche Weise zu einem Bindemittelgranulat verarbeitet und in getrocknetem Zustand mit der äusseren Phase gemischt. Diese besteht auf 0,500 kg Mg-Stearat (Ph. Eur.) und 1,500 kg Talk (Ph. Eur. II). Das ganze

Granulat wird anschliessend auf übliche Weise zu gewölbten Tabletten von 500 mg Bruttomasse und 11,5 mm Durchmesser gepresst. Anstelle dieser klassischen Tablettiermethode können auch andere Methoden zur Tablettenherstellung angewendet werden, wie Direkttablettierung sowie feste Dispersionen nach der Schmelz- und der Sprühtrocknungsmethode.

Der magensaftresistente Ueberzug kann in einem klassichen Dragierkessel aufgeleert oder aufgesprüht sowie in einer Wirbelschichtapparatur erfolgen. Zur Magenresistenz wird portionenweise eine Lösung von 2,250 kg Hydroxypropylmethylcellulosephthalat (HPMCP, Pharmacoat HP 50 ®) in einem Gemisch folgender Lösungsmittel aufgelöst: Azeton 13,00 l, Ethanol 94 Gewichtsprozent denaturiert mit 2 % Keton 13,50 l und Aqua demineralisata 2,50 l. Zu der fertigen Lösung wird als Weichmacher Rizinusöl Ph. Eur. 0,240 kg zugegeben und auf übliche Weise in Portionen auf die gewölbten Tablettenkerne aufgetragen.
Filmcoat: Nach beendeter Trocknung wird anschliessend in den gleichen Apparaturen eine Suspension folgender Zusammensetzung als Filmcoat aufgezogen: Talcum Ph. Eur. II 0,340 kg, Titan (VI)-oxyd Cronus RN 56 ® 0,400 kg, Farblack L-Rotlack 86237 N 0,324 kg, Eudragit E 12,5 % ® 4,800 kg und Polyethylenglycol 6000 pH 11 XI 0,120 kg in einem Lösungsgemisch folgender Zusammensetzung: 2-Propanol DAB 8,170 kg, Aqua demineralisata 0,200 kg Glycerintriacetat (Triacetin ®) 0,060 kg.

Beispiel 5

Herstellung von magenresistenten Filmtabletten enthaltend 67,0 mg Monoethylfumarat Ca-Salz, 30,0 mg Dimethylfumarat, 5,0 mg Monoethylfumarat Mg-Salz und 3,0 mg Monoethylfumarat Zn-Salz entsprechend 75 mg Fumarsäure ("Mite"-Tabletten)

3,000 kg Fumarsäuredimethylester, 6,700 kg Monoethylfumarat Ca-Salz, 0,500 kg Monoethylfumarat Mg-Salz sowie 0,300 kg Monoethylfumarat Zn-Salz werden zerkleinert, intensiv gemischt und mittels eines Siebes 800 homogenisiert. Dabei sollen entsprechende Schutzmassnahmen wie Atemmaske, Handschuhe, Schutzanzug etc. zur Anwendung gelangen. Hierauf wird in einem Gemisch, bestehend aus 30,000 kg Stärkederivat (STA-RX 1500 ®), 3,000 kg mikrokristalline Cellulose (Avicel pH 101 ®), 0,750 kg Polyvinylpyrolidone (PVP Kollidon ® 25), 4,000 kg Primogel ®, 0,250 kg kolloidale Kieselsäure (Aerosil ®) versetzt. Das Wirkstoffgemisch wird homogen untermischt, durch eine Sieb 200 geschlagen und mit einer 2%igen wässrigen Lösung von Polyvinylpyrolidon (K 25) auf übliche weise zu einem Bindemittelgranulat aufgearbeitet. Dem getrockneten Granulat wird eine Pulvermischung aus folgenden Hilfsstoffen als äussere Phase zugesetzt: 0,500 kg Mg-Stearat Ph. Eur. II und 0,800 kg Talk Ph. Helv. VII.

Die homogene Granulatmischung wird zu gewölbten Tablettenkernen von 500,0 mg Gewicht und 11,5 mm Durchmesser auf übliche Weise komprimiert. Neben den Bindemittelmethoden können ebenfalls andere Tablettiermethoden, gemäss den Beispielen 1 und 4, Verwendung finden.

Das Ueberziehen der Tablettenkerne mit einem magenresistenten Ueberzug sowie mit einem Filmcoat erfolgt sinngemäss wie unter den Beispielen 1 und 4 beschrieben.

Vorzugsweise werden die erfindungsgemäßen Zubereitungen peroral in Form von Tabletten oder Kapseln verabreicht, wobei diese festen Einzeldosis-Arzneiformen vorzugsweise mit einem magenresistenten Überzug versehen sind, der sich nach der Magenpassage im Dünndarmsaft im Dünndarm innerhalb weniger Minuten löst und das aktive Prinzip aus der Arzneiform freisetzt. Zum systemischen Einstieg bzw. Ausstieg ist eine niedrige Dosierung (mite) erforderlich, für die therapeutische Dosierung nach der Einstiegsphase eine höhere Dosierung (forte).

Es wurde festgestellt, daß die erfindungsgemäßen Mischpräparate nach peroraler Verabreichung eine erheblich verbesserte Wirkung gegen die verschiedensten klinischen Erscheinungsformen der Psoriasis, der psoriatischen Arthritis, der Neurodermitis sowie der Enteritis regionalis Crohn (Morbus Crohn) aufweisen.

Da in einer psoriatischen Epidermis die Aktivität de Phospholipase $A_2$ verändert ist, liegt eine mögliche Erklärung des Wirkungsmechanismus der erfindungsgemäßen Mischpräparate darin, daß dieses Enzym durch Calcium-Monoethylfumarat stimuliert wird, wobei Mg-und Zn-Kationen für den Hautstoffwechsel on Psoriasis-Patienten von großer Wichtigkeit sind.

Gegenstand der Erfindung sind neben oral verabreichbaren Präparaten in Form von Kapseln, Granulaten und Tabletten, für die kutane und transdermale Verabreichung in Form von Salben, Pflastern, Lotionen und Duschmitteln, für die parenterale Verabreichung in Form wäßriger Mikrodispersionen, O/W-Emulsionen oder öliger Lösungen für die rektale Verabreichung als Suppositorien oder Mikroklistiere sowie für die medikamentöse Behandlung von Haaren, Finger- und Zehennägeln.

Therapeutische Behandlung der Psoriasis mit dem Präparat gemäß Beispiel 4 und ihre Ergebnisse

In einer intraindividuellen Velaufsstudie über ein Jahr wurde die ambulante perorale Behandling der Psoriasis an insgesamt 24 Patienten erprobt (siehe Tabelle 1). Alle Patienten sprachen dabei vorher auf konventionelle Arzneistoffe und Therapieformen schlecht an, so daß von einer negativen Selektion gesprochen werden kann.

Die Hälfte aller peroral und ambulant behandelten Patienten zeigte objektiv eine wesentliche Besserung, welche meist erst nach mehrwöchiger Behandling eintrat.

Schwerer objektive Nebenwirkungen, insbesondere Nieren- und Leberfunktionsstörungen oder Blutbildveränderungen, wurden keine festgestellt.

Die akute Toxizität wurden vor der klinischen Prüfung an Mäusen und Ratten peroral untersucht. Die Resultate zeigten eine sehr geringe Toxizität der eingesetzten Fumarsäurederivate (siehe Tabelle 2).

Tabelle 1: Klinische Ergebnisse bei Psoriasisbehandlung

| | Studie I | Studie II |
|---|---|---|
| | n = 13 Patienten<br>Formulierung gemäß Beispiel 4 | n = 11 Patienten<br>Formulierung gemäß Beispiel 4 |
| 1. Dauer der Behandlung | 3 Monate | 1 Jahr |
| 2. Resultate: | | |
| — sehr gut | 4 Patienten | 5 Patienten |
| — gut | 3 Patienten | 1 Patient |
| — unbefriedigend | 4 Patienten | 3 Patienten |
| 3. Therapieabbruch wegen Nebenwirkungen | 2 Patienten | 2 Patienten |

Tabelle 2

| Akute Toxizitätsstudie (oral) | | | |
|---|---|---|---|
| | Geschlecht | Zusammensetzung gemäss Beispiel 4 | |
| | | Mänser | Ratte |
| $LD_{50}$ (24 h) in mg/kg | männlich<br>weiblich | 5750<br>8200 | 4700<br>4600 |
| $LD_{50}$ (14 Tage) in mg/kg | männlich<br>weiblich | 5600<br>6950 | 4700<br>3900 |
| "Lowest Toxical Dose" in mg/kg | männlich<br>weiblich | 3160<br>3160 | 3160<br>3160 |
| "Lowest Lethal Dose" in mg/kg | | 5620 | 4640 |
| Magen und Darm Schleimhautblutung in mg/kg | | 5600 | kein |
| Milzentzündung | | kein | kein |
| Epithel Ödeme | | kein | kein |

Im folgenden wird die Behandlung von Patienten mit Neurodermitis und Enteritis regionalis Crohn (Morbus Crohn) und ihr therapeutisches Ergebnis beschrieben:

HM - weiblich - 1951

- Krankheit: Enteritis regionalis Crohn
- Dosis: 3 Tabletten/Tag (Formulierung gemäß Beispiel 5)
- Dauer: 7.5.86 - 27.11.86
- Erfolg: Darm seither ruhig ohne Medikation

EL - weiblich - 1919

- Krankheit: Enteritis regionalis Crohn
- Therapiebeginn: 24.3.84
- Dosis: 3 Tabletten/Tag (Formulierung gemäß Beispiel 5)
- Erfolg: Darmblutungen und Geschwüre zur Ruhe gekommen

WCh - weiblich - 1945

- Krankheit: Enteritis regionalis Crohn
- Therapiebeginn: 9.4.1988
- Dosis: 3 Tabletten/Tag (Formulierung gemäß Beispiel 5)
- Erfolg: alles ruhiger, aber noch zu früh zum beurteilen

NK - männlich - 1971

- Krankheit: Neurodermitis
- Dauer der Krankheit: 16 Jahre
- Therapiebeginn: 16.12.87
- Dosis: 3 Tabletten/Tag (Formulierung gemäß Beispiel 4)
- Erfolg: sehr gut

DN - weiblich - 1926

- Krankheit: Neurodermitis
- Dauer der Krankheit: 40 Jahre
- Therapiebeginn: 4.9.86

- Dosis: 2 Tabletten/Tag (Formulierung gemäß Beispiel 4)
- Erfolg: gut

AS - weiblich - 1941

- Krankheit: Neurodermitis
- Dauer der Krankheit: 20 Jahre
- Therapiebeginn: 16.12.87
- Dosis: 6 Tabletten/Tag (Formulierung gemäß Beispiel 5)
- Erfolg: sehr gut

D.E. - weiblich - 1960

- Krankheit: Ichthyosis
- Therapiebeginn: 15.3.1988
- Dosis: 6 Tabletten/Tag (Formulierung gemäß Beispiel 5)
- Erfolg: gut

**Patentansprüche**
**Patentansprüche für folgende Vertragsstaaten : AT, BE, CH, DE, FR, GB, IT, LI, LU, NL, SE**

1. Verwendung einer oder mehrerer Verbindungen aus der Gruppe der Calcium-, Magnesium-, Zink- und Eisensalze von Fumarsäuremonoalkylester der allgemeinen Formel

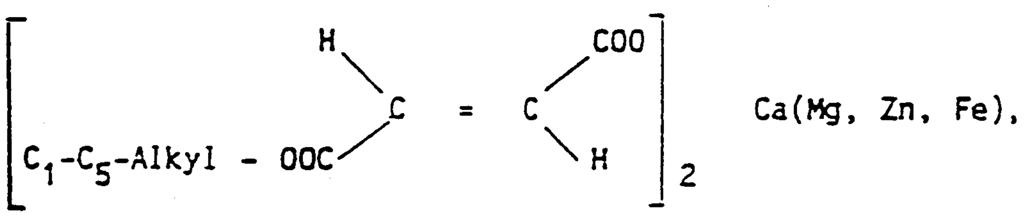

gegebenenfalls im Gemisch mit Dialkylfumarat der Formel

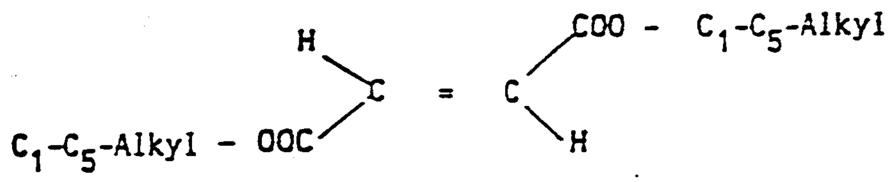

und übliche pharmazeutische Hilfs- und Trägerstoffe zur Herstellung einer pharmazeutischen Zubereitung zur Behandlung der psoriatischen Arthritis, Neurodermitis und Enteritis regionalis Crohn.

2. Verwendung nach Anspruch 1, dadurch gekennzeichnet, daß es sich um das Calciumsalz des Fumarsäuremonoethylesters handelt.

3. Verwendung nach Anspruch 1, dadurch gekennzeichnet, daß es sich um das Calciumsalz des Fumarsäuremonomethylesters handelt.

4. Verwendung nach Anspruch 1, dadurch gekennzeichnet, daß es sich um das Calciumsalz des Fumarsäuremonoethylesters im Gemisch mit Dimethylfumarat handelt.

5. Verwendung nach Anspruch 1, dadurch gekennzeichnet, daß es sich um das Calcium- und Zinksalz des Fumarsäuremonomethylesters im Gemisch mit Dimethylfumarat handelt.

6. Verwendung nach Anspruch 1, dadurch gekennzeichnet, daß es sich um das Gemisch von Calcium-, Magnesium- und Zinksalz des Fumarsäuremonoethylesters im Gemisch mit Dimethylfumarat handelt.

7. Verwendung nach Anspruch 1 zur Herstellung einer pharmazeutischen Zubereitung zur oralen Verabreichung in Form von Tabletten oder Kapseln, dadurch gekennzeichnet, daß es sich um das Calciumsalz des Fumarsäuremonoalkylesters in einer Menge von 100 bis 300 mg, wobei das Gesamtgewicht der Wirkstoffe 100 bis 300 mg beträgt, handelt.

8. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 290 Gewichtsteile des Calciumsalzes des Fumarsäuremonoalkylesters und 290 bis 10 Gewichtsteile Dimethylfumarat, wobei das Gesamtgewicht der Wirkstoffe 100 bis 300 mg beträgt, handelt.

9. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters, wobei das Gesamtgewicht der Wirkstoffe 100 bis 300 mg beträgt, handelt.

10. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 250 Gewichtsteile des Calciumsalzes des Fumarsäuremonoalkylester, 250 bis 10 Gewichtsteile Dimethylfumarat, 1 bis 50 Gewichtsteile des Magnesiumsalzes des Fumarsäuremonoalkylesters und 1 bis 50 Gewichtsteile des Zinksalzes des Fumarsäuremonoalkylesters, wobei das Gesamtgewicht der Wirkstoffe 100 bis 300 mg beträgt, handelt.

11. Verwendung nach einem der vorhergehenden Ansprüche zur Herstellung einer pharmazeutischen Zubereitung zur oralen Verabreichung, dadurch gekennzeichnet, daß sie mit einem magensaftresistenten Überzug versehen sind.

12. Verwendung nach einem der vorhergehenden Ansprüche zur Herstellung einer pharmazeutischen Zubereitung zur Behandlung psoriatischen Arthritis, Neurodermitis und Enteritis regionalis Crohn für die perorale Verabreichung in Form von Kapseln, Granulaten und Tabletten, für die kutane und transdermale Verabreichung in Form von Salben, Pflastern, Lotionen und Duschmitteln, für die parenterale Verabreichung in Form wäßriger Mikro-Dispersionen, O/W-Emulsionen oder öligen Lösungen, für die rektale Verabreichung in Form von Suppositorien oder Mikroklistiere, sowie für die medikamentöse Behandlung von Haaren, Finger- und Zehennägeln.

13. Pharmazeutische Zubereitung zur Behandlung der Psoriasis, dadurch gekennzeichnet, daß sie eine oder mehrere Verbindungen aus der Gruppe der Calcium-, Magnesium-, Zink- und Eisensalze von Fumarsäuremonomethylester der allgemeinen Formel

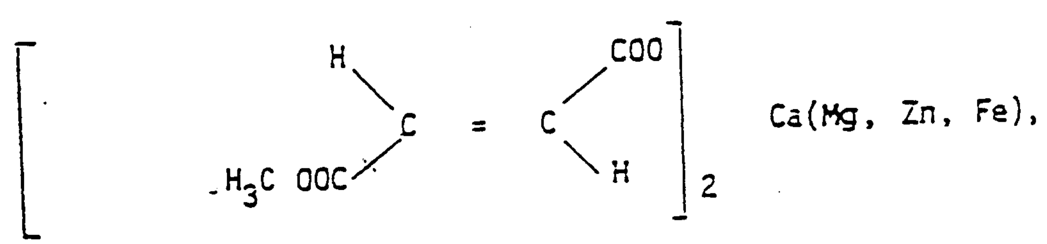

gegebenfalls im Gemisch mit Dialkylfumarat der Formel:

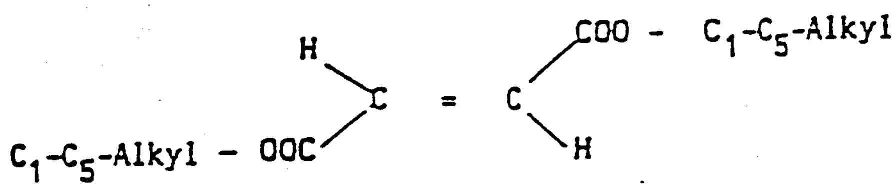

und Trägerstoffe enthält, wobei die Zubereitungen Fumarsäure nicht enthalten.

**14.** Pharmazeutische Zubereitung nach Anspruch 13, dadurch gekennzeichnet, daß sie das Calciumsalz des Fumarsäuremonomethylesters enthält.

**15.** Pharmazeutische Zubereitungsform zur oralen Verabreichung in Form von Tabletten oder Kapseln nach Anspruch 13, dadurch gekennzeichnet, daß sie das Calciumsalz des Fumarsäuremonomethylesters in einer Menge von 100 bis 300 mg enthält, wobei das Gesamtgewicht der Wirkstoffe 100 bis 300 mg beträgt.

**16.** Pharmazeutische Zubereitungsform zur oralen Verabreichung in Form von Tabletten oder Kapseln nach Anspruch 13, dadurch gekennzeichnet, daß sie 10 bis 290 Gewichtsteile des Calciumsalzes des Fumarsäuremonomethylesters und 290 bis 10 Gewichtsteile Dimethylfumarat enthält, wobei das Gesamtgewicht der Wirkstoffe 100 bis 300 mg beträgt.

**17.** Pharmazeutische Zubereitungsform zur oralen Verabreichung in Form von Tabletten oder Kapseln nach Anspruch 13, dadurch gekennzeichnet, daß sie 10 bis 250 Gewichtsteile des Calciumsalzes des Fumarsäuremonomethylesters, 1 bis 50 Gewichtsteile Dimethylfumarat und 1 bis 50 Gewichtsteile des Zinksalzes des Fumarsäuremonomethylesters enthält, wobei das Gesamtgewicht der Wirkstoffe 100 bis 300 mg beträgt.

**18.** Pharmazeutische Zubereitungsform zur oralen Verabreichung in Form von Tabletten oder Kapseln nach Anspruch 13, dadurch gekennzeichnet, daß sie 10 bis 250 Gewichtsteile des Calciumsalzes des Fumarsäuremonomethylesters, 250 bis 10 Gewichtsteile Dimethylfumarat, 1 bis 50 Gewichtsteile des Magnesiumsalzes des Fumarsäuremonomethylesters und 1 bis 50 Gewichtsteile des Zinksalzes des Fumarsäuremonomethylesters enthält, wobei das Gesamtgewicht der Wirkstoffe 100 bis 300 mg beträgt.

**19.** Pharmazeutische Zubereitungsform zur oralen Verabreichung nach einem der Ansprüche 13 bis 18, dadurch gekennzeichnet, daß sie mit einem magensaftresistenten Überzug versehen sind.

**20.** Pharmazeutische Zubereitungen zur Behandlung der Psoriasis gemäß Anspruch 13 für die perorale Verabreichung in Form von Kapseln, Granulaten und Tabletten, für die kutane und transdermale Verabreichung in Form von Salben, Pflastern, Lotionen und Duschmitteln, für die parenterale Verabreichung in Form wäßriger Mikro-Dispersionen, O/W-Emulsionen oder Mikroklistieren, sowie für die medikamentöse Behandlung von Haaren, Finger- und Zehennägeln.

**Patentansprüche für folgende Vertragsstaaten : ES, GR**

**1.** Verwendung einer oder mehrerer Verbindungen aus der Gruppe der Calcium-, Magnesium-, Zink- und Eisensalze von Fumarsäuremonoalkylester der allgemeinen Formel

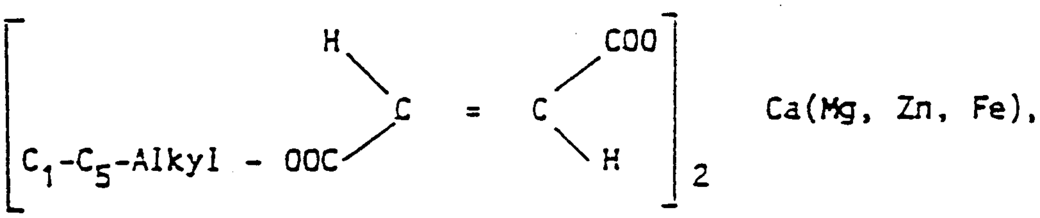

gegebenenfalls im Gemisch mit Dialkylfumarat der Formel

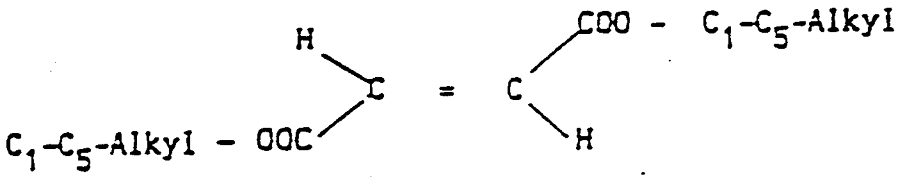

und übliche pharmazeutische Hilfs- und Trägerstoffe zur Herstellung einer pharmazeutischen Zubereitung zur Behandlung der psoriatischen Arthritis, Neurodermitis und Enteritis regionalis Crohn.

2. Verwendung nach Anspruch 1, dadurch gekennzeichnet, daß es sich um das Calciumsalz des Fumarsäuremonoethylesters handelt.

3. Verwendung nach Anspruch 1, dadurch gekennzeichnet, daß es sich um das Calciumsalz des Fumarsäuremonomethylesters handelt.

4. Verwendung nach Anspruch 1, dadurch gekennzeichnet, daß es sich um das Calciumsalz des Fumarsäuremonoethylesters im Gemisch mit Dimethylfumarat handelt.

5. Verwendung nach Anspruch 1, dadurch gekennzeichnet, daß es sich um das Calcium- und Zinksalz des Fumarsäuremonomethylesters im Gemisch mit Dimethylfumarat handelt.

6. Verwendung nach Anspruch 1, dadurch gekennzeichnet, daß es sich um das Gemisch von Calcium-, Magnesium- und Zinksalz des Fumarsäuremonoethylesters im Gemisch mit Dimethylfumarat handelt.

7. Verwendung nach Anspruch 1 zur Herstellung einer pharmazeutischen Zubereitung zur oralen Verabreichung in Form von Tabletten oder Kapseln, dadurch gekennzeichnet, daß es sich um das Calciumsalz des Fumarsäuremonoalkylesters in einer Menge von 100 bis 300 mg, wobei das Gesamtgewicht der Wirkstoffe 100 bis 300 mg beträgt, handelt.

8. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 290 Gewichtsteile des Calciumsalzes des Fumarsäuremonoalkylesters und 290 bis 10 Gewichtsteile Dimethylfumarat, wobei das Gesamtgewicht der Wirkstoffe 100 bis 300 mg beträgt, handelt.

9. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 250 Gewichtsteile des Calciumsalzes des Fumarsäuremonoalkylesters, 1 bis 50 Gewichtsteile Dimethylfumarat und 1 bis 50 Gewichtsteile des Zinksalzes des Fumarsäuremonoalkylesters, wobei das Gesamtgewicht der Wirkstoffe 100 bis 300 mg beträgt, handelt.

10. Verwendung nach Anspruch 1 zur Herstellung einer pharmazeutischen Zubereitung zur oralen Verabreichung in Form von Tabletten oder Kapseln, dadurch gekennzeichnet, daß es sich um 10 bis 250 Gewichtsteile des Calciumsalzes des Fumarsäuremonoalkylester, 250 bis 10 Gewichtsteile Dimethylfumarat, 1 bis 50 Gewichtsteile des Magnesiumsalzes des Fumarsäuremonoalkylesters und 1 bis 50

Gewichtsteile des Zinksalzes des Fumarsäuremonoalkylesters, wobei das Gesamtgewicht der Wirkstoffe 100 bis 300 mg beträgt, handelt.

11. Verwendung nach einem der vorhergehenden Ansprüche zur Herstellung einer pharmazeutischen Zubereitung zur oralen Verabreichung, dadurch gekennzeichnet, daß sie mit einem magensaftresistenten Überzug versehen sind.

12. Verwendung nach einem der vorhergehenden Ansprüche zur Herstellung einer pharmazeutischen Zubereitung zur Behandlung psoriatischen Arthritis, Neurodermitis und Enteritis regionalis Crohn für die perorale Verabreichung in Form von Kapseln, Granulaten und Tabletten, für die kutane und transdermale Verabreichung in Form von Salben, Pflastern, Lotionen und Duschmitteln, für die parenterale Verabreichung in Form wäßriger Mikro-Dispersionen, O/W-Emulsionen oder öligen Lösungen, für die rektale Verabreichung in Form von Suppositorien oder Mikroklistiere, sowie für die medikamentöse Behandlung von Haaren, Finger- und Zehennägeln.

13. Verfahren zur Herstellung einer pharmazeutischen Zubereitung zur Behandlung der Psoriasis, dadurch gekennzeichnet, daß man eine oder mehrere Verbindungen aus der Gruppe der Calcium-, Magnesium-, Zink- und Eisensalze von Fumarsäuremonomethylester der allgemeinen Formel

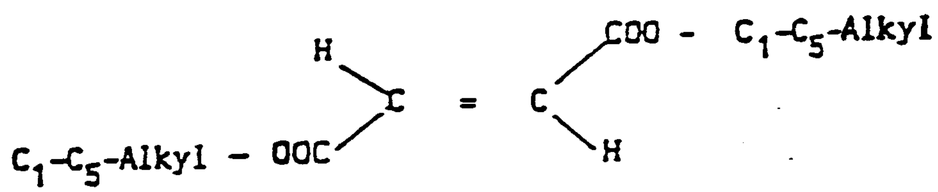

gegebenenfalls im Gemisch mit Dialkylfumarat der Formel:

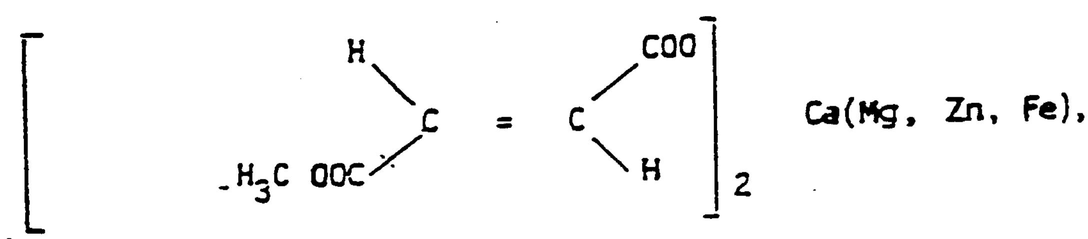

mit Trägerstoffen mischt, wobei die Zubereitungen Fumarsäure nicht enthalten.

14. Verfahren zur Herstellung einer pharmazeutischen Zubereitung nach Anspruch 13, dadurch gekennzeichnet, daß man das Calciumsalz des Fumarsäuremonomethylesters verwendet.

15. Verfahren zur Herstellung einer pharmazeutischen Zubereitungsform zur oralen Verabreichung in Form von Tabletten oder Kapseln nach Anspruch 13, dadurch gekennzeichnet, daß man das Calciumsalz des Fumarsäuremonomethylesters in einer Menge von 100 bis 300 mg verwendet, wobei das Gesamtgewicht der Wirkstoffe 100 bis 300 mg beträgt.

16. Verfahren zur Herstellung einer pharmazeutischen Zubereitungsform zur oralen Verabreichung in Form von Tabletten oder Kapseln nach Anspruch 13, dadurch gekennzeichnet, daß man 10 bis 290 Gewichtsteile des Calciumsalzes des Fumarsäuremonomethylesters und 290 bis 10 Gewichtsteile Dimethylfumarat verwendet, wobei das Gesamtgewicht der Wirkstoffe 100 bis 300 mg beträgt.

17. Verfahren zur Herstellung einer pharmazeutischen Zubereitungsform zur oralen Verabreichung in Form von Tabletten oder Kapseln nach Anspruch 13, dadurch gekennzeichnet, daß man 10 bis 250 Gewichtsteile des Calciumsalzes des Fumarsäuremonomethylesters, 1 bis 50 Gewichtsteile Dimethylfumarat und 1 bis 50 Gewichtsteile des Zinksalzes des Fumarsäuremonomethylesters verwendet, wobei das Gesamtgewicht der Wirkstoffe 100 bis 300 mg beträgt.

**18.** Verfahren zur Herstellung einer pharmazeutischen Zubereitungsform zur oralen Verabreichung in Form von Tabletten oder Kapseln nach Anspruch 13, dadurch gekennzeichnet, daß man 10 bis 250 Gewichtsteile des Calciumsalzes des Fumarsäuremonomethylesters, 250 bis 10 Gewichtsteile Dimethylfumarat, 1 bis 50 Gewichtsteile des Magnesiumsalzes des Fumarsäuremonomethylesters und 1 bis 50 Gewichtsteile des Zinksalzes des Fumarsäuremonomethylesters verwendet, wobei das Gesamtgewicht der Wirkstoffe 100 bis 300 mg beträgt.

**19.** Verfahren zur Herstellung einer pharmazeutischen Zubereitungsform zur oralen Verabreichung nach einem der Ansprüche 13 bis 18, dadurch gekennzeichnet, daß man sie mit einem magensaftresistenten Überzug versieht.

**20.** Verfahren zur Herstellung einer pharmazeutischen Zubereitung zur Behandlung der Psoriasis gemäß Anspruch 13 für die perorale Verabreichung in Form von Kapseln, Granulaten und Tabletten, für die kutane und transdermale Verabreichung in Form von Salben, Pflastern, Lotionen und Duschmitteln, für die parenterale Verabreichung in Form wäßriger Mikro-Dispersionen, O/W-Emulsionen oder Mikroklistieren, sowie für die medikamentöse Behandlung von Haaren, Finger- und Zehennägeln.

**Claims**

**Claims for the following Contracting States : AT, BE, CH, DE, FR, GB, IT, LI, LU, NL, SE**

**1.** Use of one or more compounds from the group formed by the calcium, magnesium, zinc and iron salts of fumaric acid monoalkyl ester having the general Formula

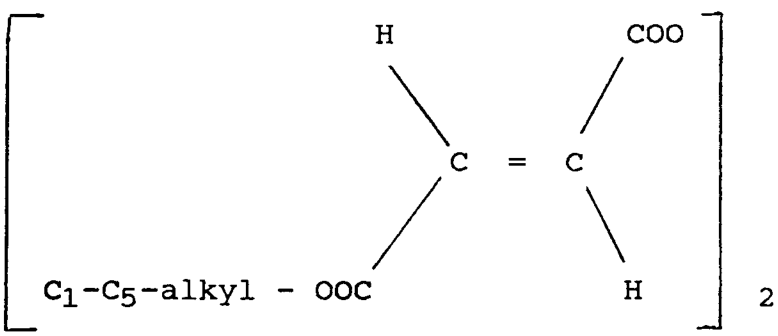

Ca(Mg, Zn, Fe),

possibly mixed with dialkyl fumarate having the Formula

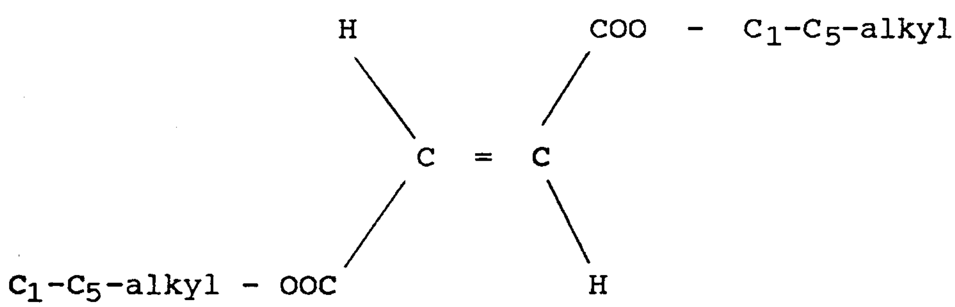

and usual pharmaceutical ancillary and vehicling substances, for the production of a pharmaceutical preparation for the treatment of psoriatic arthritis, neurodermitis and enteritis regionalis Crohn.

**2.** Use according to claim 1, characterized in that the calcium salt of fumaric acid monoethyl ester is used.

3. Use according to claim 1, characterized in that the calcium salt of fumaric acid monomethyl ester is used.

4. Use according to claim 1, characterized in that the calcium salt of fumaric acid monoethyl ester is used mixed with dimethyl fumarate.

5. Use according to claim 1, characterized in that the calcium and zinc salt of fumaric acid monomethyl ester is used mixed with dimethyl fumarate.

6. Use according to claim 1, characterized in that the mixture of calcium, magnesium and zinc salt of fumaric acid monoethyl ester is used mixed with dimethyl fumarate.

7. Use according to claim 1 for the production of a pharmaceutical preparation for oral administration in the form of tablets or capsules, characterized in that the calcium salt of fumaric acid monoalkyl ester is used in a quantity of 100 to 300 mg, the total weight of the active substances being 100 to 300 mg.

8. Use according to claim 1 for the production of a pharmaceutical preparation for oral administration in the form of tablets or capsules, characterized in that 10 to 290 parts by weight of the calcium salt of fumaric acid monoalkyl ester and 290 to 10 parts by weight of dimethyl fumarate are used, the total weight of the active substances being 100 to 300 mg.

9. Use according to claim 1 for the production of a pharmaceutical preparation for oral administration in the form of tablets or capsules, characterized in that 10 to 250 parts by weight of the calcium salt of fumaric acid monoalkyl ester, 1 to 50 parts by weight dimethyl fumarate and 1 to 50 parts by weight of the zinc salt of fumaric acid monoalkyl ester are used, the total weight of the active substances being 100 to 300 mg.

10. Use according to claim 1 for the production of a pharmaceutical preparation for oral administration in the form of tablets or capsules, characterized in that 10 to 250 parts by weight of the calcium salt of fumaric acid monoalkyl ester, 250 to 10 parts by weight dimethyl fumarate, 1 to 50 parts by weight of the magnesium salt of fumaric acid monoalkyl ester and 1 to 50 parts by weight of the zinc salt of fumaric acid monoalkyl ester are used, the total weight of the active substances being 100 to 300 mg.

11. Use according to one of the preceding claims for the production of a pharmaceutical preparation for oral administration, characterized in that it has a coating resistant to stomach juices.

12. Use according to one of the preceding claims for the production of a pharmaceutical preparation for the treatment of psoriatic arthritis, neurodermitis and enteritis regionalis Crohn, for oral administration in the form of capsules, granulates and tablets, for cutaneous and transdermal administration in the form of ointments, plasters, lotions and spraying agents, for parenteral administration in the form of aqueous microdispersions, O/W emulsions or oily solutions, for rectal administration in the form of suppositories or micro enemas, and also for the medicamentous treatment of hair, finger nails and toe nails.

13. A pharmaceutical preparation for the treatment of psoriasis, characterized in that it contains one or more compounds from the group formed by the calcium, magnesium, zinc and iron salts of fumaric acid monoethyl ester having the general Formula

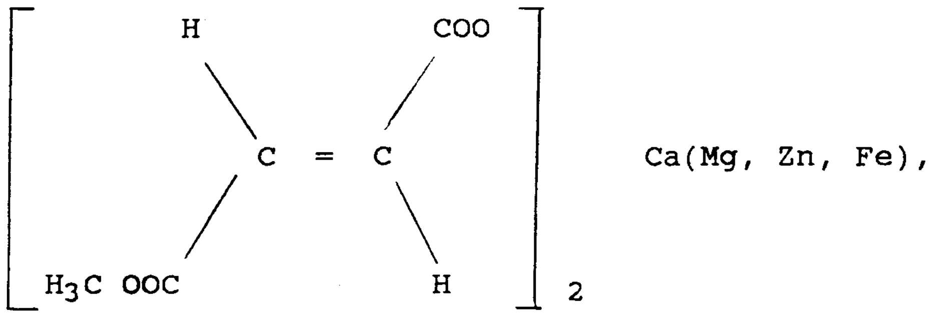

possibly mixed with dialkyl fumarate having the Formula:

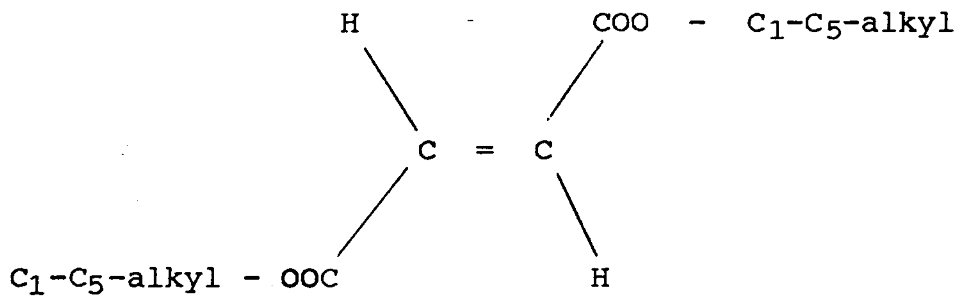

and vehicling substances, wherein the preparations do not contain fumaric acid.

**14.** A pharmaceutical form of preparation according to claim 13, characterized in that it contains the calcium salt of fumaric acid monomethyl ester.

**15.** A pharmaceutical form of preparation for oral administration in the form of tablets or capsules according to claim 13, characterized in that it contains the calcium salt of fumaric acid monomethyl ester in a quantity of 100 to 300 mg, the total weight of the active substances being 100 to 300 mg.

**16.** A pharmaceutical form of preparation for oral administration in the form of tablets or capsules according to claim 13, characterized in that it contains 10 to 290 parts by weight of the calcium salt of fumaric acid monomethyl ester and 290 to 10 parts by weight dimethyl fumarate, the total weight of the active substances being 100 to 300 mg.

**17.** A pharmaceutical form of preparation for oral administration in the form of tablets or capsules according to claim 13, characterized in that it contains 10 to 250 parts by weight of the calcium salt of fumaric acid monomethyl ester, 1 to 50 parts by weight dimethyl fumarate and 1 to 50 parts by weight of the zinc salt of fumaric acid monomethyl ester, the total weight of the active substances being 100 to 300 mg.

**18.** A pharmaceutical form of preparation for oral administration in the form of tablets or capsules according to claim 13, characterized in that it contains 10 to 250 parts by weight of the calcium salt of fumaric acid monomethyl ester, 250 to 10 parts by weight dimethyl fumarate, 1 to 50 parts by weight of the magnesium salt of fumaric acid monomethyl ester and 1 to 50 parts by weight of the zinc salt of fumaric acid monomethyl ester, the total weight of the active substances being 100 to 300 mg.

**19.** A pharmaceutical form of preparation for oral administration according to one of claims 13 to 18, characterized in that it has a coating resistant to stomach juices.

**20.** Pharmaceutical preparations for the treatment of psoriosis according to claim 13, for oral administration in the form of capsules, granulates and tablets, for cutaneous and transdermal administration in the

form of ointments, plasters, lotions and spraying agents, for parenteral administration in the form of aqueous microdispersions, O/W emulsions or micro enemas and also for the medicamentous treatment of hair, finger nails and toe nails.

**Claims for the following Contracting States : ES, GR**

1. The use of one or several compounds selected from the group formed by the calcium, magnesium, zinc and iron salts of fumaric acid monoalkyl ester having the general formula

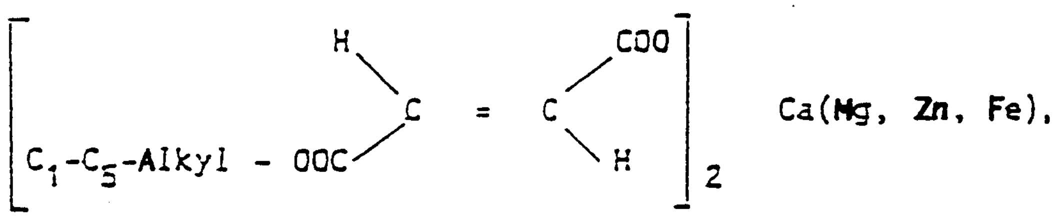

optionally mixed with dialkyl fumarate having the formula

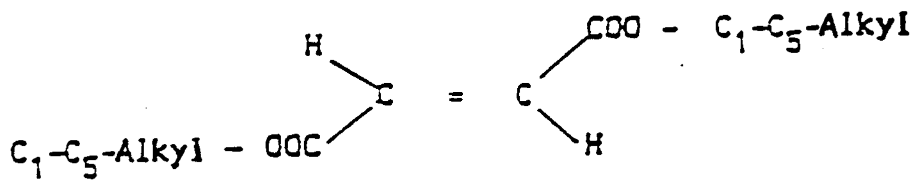

and the usual pharmaceutical ancillary and carrier substances for the production of a pharmaceutical preparation for the treatment of psoriatic arthritis, neurodermitis and enteritis regionalis Crohn.

2. The use according to claim 1, characterised in that the calcium salt of fumaric acid monoethyl ester is used.

3. The use according to claim 1, characterised in that the calcium salt of fumaric monomethyl ester is used.

4. The use according to claim 1, characterised in that the calcium salt of fumaric acid monoethyl ester is used in a mixture with dimethyl fumarate.

5. The use according to claim 1, characterised in that the calcium and zinc salt of fumaric acid monomethyl ester is used in a mixture with dimethyl fumarate.

6. The use according to claim 1, characterised in that a mixture of calcium, magnesium and zinc salt of fumaric acid monoethyl ester in a mixture with dimethyl fumarate is used.

7. The use according to claim 1 for the production of a pharmaceutical preparation for oral administration in the form of tablets or capsules, characterised in that the calcium salt of fumaric acid monoalkyl ester in the amount to 100 to 300 mg is used, the total weight of the active ingredients being 100 to 300 mg.

8. The use according to claim 1 for the production of a pharmaceutical preparation for oral administration in the form of tablets or capsules, characterised in that 10 to 290 parts by weight of the calcium salt of fumaric monoalkyl ester and 290 to 10 parts by weight of dimethyl fumarate are used, the total weight of the active substances being 100 to 300 mg.

9. The use according to claim 1 for the production of a pharmaceutical preparation for the oral administration in the form of tablets or capsules, characterised in that 10 to 250 parts by weight of the calcium salt of fumaric acid monoalkyl ester, 1 to 50 parts by weight dimethyl fumarate and 1 to 50 parts by weight of the zinc salt of fumaric acid monoalkyl ester is used, the total weight of the active

substances being 100 to 300 mg.

**10.** The use according to claim 1 for the production of a pharmaceutical preparation for oral administration in the form of tablets or capsules, characterised in that 10 to 250 parts by weight of the calcium salt of the fumaric acid monoalkyl ester, 250 to 10 parts by weight dimethyl fumarate, 1 to 50 parts by weight of the magnesium salt of the fumaric acid monoalkylester and 1 to 50 parts by weight of the zinc salt of the fumaric acid monoalkyl ester are used, the total weight of the active substances being 100 to 300 mg.

**11.** The use according to any of the preceding claims for the production of a pharmaceutical preparation for oral administration, characterised in that there is provided a coating resistant to gastric juices.

**12.** The use according to any of the preceding claims for the production of a pharmaceutical preparation for treating psoriatic arthritis, neurodermitis and enteritis regionalis Crohn for peroral administration in the form of capsules, granulates and tablets, for cutaneous and transdermal administration in the form of ointments, plasters, lotions and spraying agents, for parenteral administration in the form of aqueous micro-dispersions, O/W emulsions or oily solutions, for rectal administration in the form of suppositories or micro-enemas as well as for the medicamentous treatment of hair, fingernails and toenails.

**13.** A process for the production of a pharmaceutical preparation for treating psoriasis, characterised in that one or several compounds selected from the group of calcium, magnesium, zinc and iron salts of fumaric acid monomethyl ester having the general formula

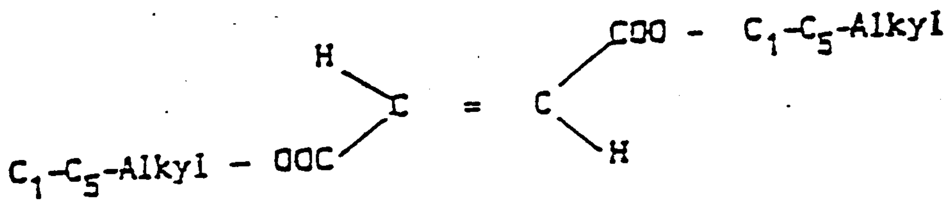

optionally mixed with dialkyl fumarate having the formula

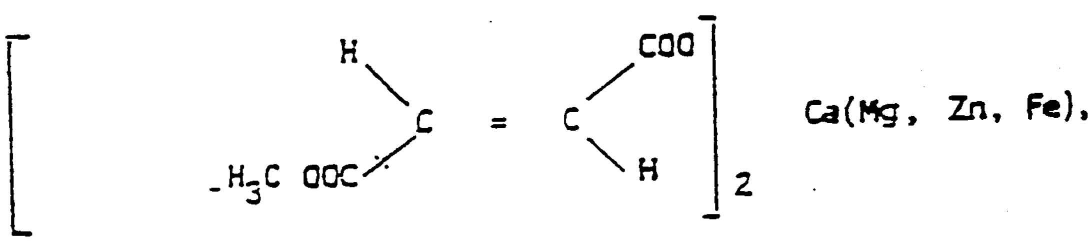

are mixed with carrier substances, said preparations not containing fumaric acid.

**14.** A process for the production of a pharmaceutical preparation according to claim 13, characterised in that the calcium salt of fumaric acid monomethyl ester is used.

**15.** A process for the production of a pharmaceutical form of preparation for oral administration in the form of tablets or capsules according to claim 13, characterised in that the calcium salt of fumaric acid monomethyl ester is used in an amount of 100 to 300 mg, the total weight of the active substances being 100 to 300 mg.

**16.** A process for the production of a pharmaceutical form of preparation for oral administration in the form of tablets or capsules according to claim 13, characterised in that 10 to 290 parts by weight of the calcium salt of fumaric acid monomethyl ester and 290 to 10 parts by weight of dimethyl fumarate are used, the total weight of the active substances being 100 to 300 mg.

17. A process for the production of a pharmaceutical form of preparation for oral administration in the form of tablets or capsules according to claim 13, characterised in that 10 to 250 parts by weight of the calcium salt of fumaric acid monomethyl ester, 250 to 10 parts by weight dimethyl fumarate, 1 to 50 parts by weight of the magnesium salt of the fumaric acid monomethyl ester and 1 to 50 parts by weight of the zinc salt of the fumaric acid are used, the total weight of the active substances being 100 to 300 mg.

18. A process for the production of a pharmaceutical form of preparation for oral administration in the form of tablets or capsules according to claim 13, characterised in that 10 to 250 parts by weight of the calcium salt of fumaric acid monomethyl ester, 250 to 10 parts by weight of dimethyl fumarate, 1 to 50 parts by weight of the magnesium salt of fumaric acid monomethyl ester and 1 to 50 parts by weight of the zinc salt of fumaric acid monomethyl ester are used, the total weight of the active substances being 100 to 300 mg.

19. A process for the production of a pharmaceutical form of preparation for oral administration according to any of the claims 13 to 18, characterised in that there is provided a coating resistant to gastric juices.

20. A process for the production of a pharmaceutical preparation for treating psoriasis according to claim 13 for the peroral administration in the form of capsules, granulates and tablets, for the cutaneous and transdermal administration in the form of ointments, plasters, lotions and spraying agents, for the parenteral administration in the form of aqueous micro-dispersions, O/W emulsions or microenemas as well as for the medicamentous treatment of hair, fingernails and toenails.

**Revendications**

**Revendications pour les Etats contractants suivants : AT, BE, CH, DE, FR, GB, IT, LI, LU, NL, SE**

1. Utilisation d'un ou plusieurs composés du groupe des sels de calcium, de magnésium, de zinc et de fer d'ester monoalkylique d'acide fumarique répondant à la formule générale

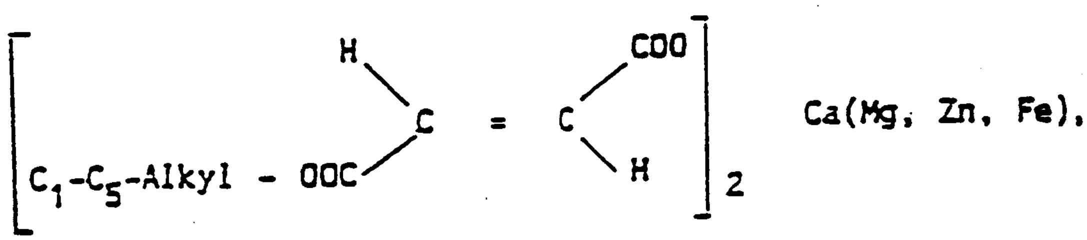

éventuellement en mélange avec du fumarate de dialkyle de formule

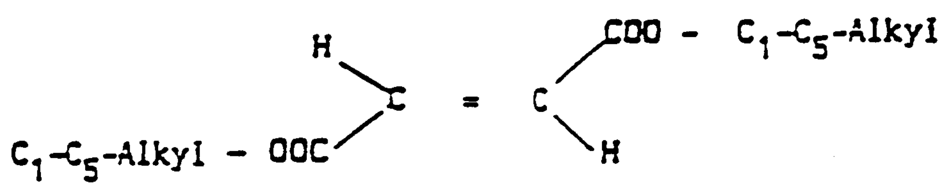

et des adjuvants et substances de support pharmaceutiques usuels pour la fabrication d'une préparation pharmaceutique pour le traitement de l'arthrite psoriasique, de la neurodermite et de l'iléite régionale de Crohn.

2. Utilisation selon la revendication 1, caractérisée en ce qu'il s'agit du sel de calcium de l'ester monoéthylique de l'acide fumarique.

3. Utilisation selon la revendication 1, caractérisée en ce qu'il s'agit du sel de calcium de l'ester monométhylique de l'acide fumarique.

4. Utilisation selon la revendication 1, caractérisée en ce qu'il s'agit du sel de calcium d'ester monoéthylique de l'acide fumarique en mélange avec du fumarate de diméthyle.

5. Utilisation selon la revendication 1, caractérisée en ce qu'il s'agit du sel de calcium et du sel de zinc d'ester monométhylique de l'acide fumarique en mélange avec du fumarate de diméthyle.

6. Utilisation selon la revendication 1, caractérisée en ce qu'il s'agit du mélange de sels de calcium, de magnésium et de zinc d'ester monoéthylique de l'acide fumarique en mélange avec du fumarate de diméthyle.

7. Utilisation selon la revendication 1 pour la fabrication de préparations pharmaceutiques pour l'administration orale sous forme de tablettes ou de capsules, caractérisée en ce qu'il s'agit du sel de calcium d'ester monoalkylique de l'acide fumarique en une quantité de 100 à 300 mg, le poids total des substances actives étant de 100 à 300 mg.

8. Utilisation selon la revendication pour la fabrication d'une préparation pharmaceutique pour l'administration orale sous forme de tablettes ou de capsules, caractérisée en ce qu'il s'agit de 10 à 290 parties en poids du sel de calcium d'ester monoalkylique de l'acide fumarique et 290 à 10 parties en poids de fumarate de diméthyle, le poids total des substances actives étant de 100 à 300 mg.

9. Utilisation selon la revendication 1 pour la fabrication d'une préparation pharmaceutique pour l'administration orale sous forme de tablettes ou de capsules, caractérisée en ce qu'il s'agit de 10 à 250 parties en poids du sel de calcium d'ester monoalkylique de l'acide fumarique, 1 à 50 parties en poids de fumarate de diméthyle et 1 à 50 parties en poids du sel de zinc d'ester monoalkylique de l'acide fumarique, le poids total des substances actives étant de 100 à 300 mg.

10. Utilisation selon la revendication 1 pour la fabrication d'une préparation pharmaceutique pour l'administration orale sous forme de tablettes ou de capsules, caractérisée en ce qu'il s'agit de 10 à 250 parties en poids du sel de calcium d'ester monoalkylique de l'acide fumarique, 250 à 10 parties en poids de fumarate de diméthyle, 1 à 50 parties en poids du sel de magnésium d'ester monoalkylique de l'acide fumarique et 1 à 50 parties en poids du sel de zinc d'ester monoalkylique de l'acide fumarique, le poids total des substances actives étant de 100 à 300 mg.

11. Utilisation selon l'une des revendications précédentes pour la fabrication d'une préparation pharmaceutique pour administration orale, caractérisée en ce qu'elle est pourvue d'un revêtement résistant au suc gastrique.

12. Utilisation selon l'une des revendications précédentes pour la fabrication d'une préparation pharmaceutique pour le traitement de l'arthrite psoriasique, de la neurodermite et de l'iléite régionale de Crohn, pour l'administration orale sous forme de capsules, granulés et tablettes, pour l'administration dermique et transdermique sous forme de pommades, emplâtres, lotions et produits de douche, pour l'administration parentérale sous forme de microdispersions aqueuses, d'émulsions huile/eau ou d'émulsions huileuses, pour l'administration rectale sous forme du suppositoires ou de microclystères, ainsi que pour le traitement médicamenteux des cheveux et des ongles des doigts et des orteils.

13. Préparation pharmaceutique pour le traitement du psoriasis, caractérisée en ce qu'elle contient un ou plusieurs composés du groupe des sels de calcium, de magnésium, de zinc et de fer d'ester monométhylique de l'acide fumarique répondant à la formule générale

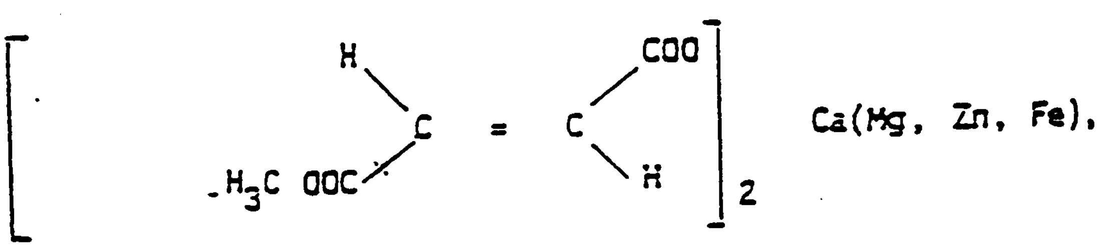

éventuellement en mélange avec du fumarate de dialkyle de formule:

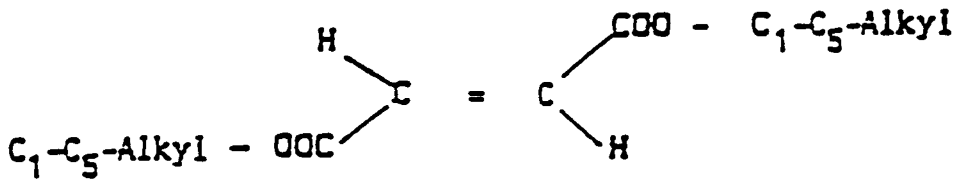

et des substances de support, les préparations ne contenant pas d'acide fumarique.

**14.** Préparation pharmaceutique selon la revendication 13, caractérisée en ce qu'elle contient le sel de calcium d'ester monométhylique de l'acide fumarique.

**15.** Forme de préparation pharmaceutique pour l'administration orale sous forme de tablettes ou de capsules selon la revendication 13, caractérisée en ce qu'elle contient le sel de calcium d'ester monométhylique de l'acide fumarique à raison de 100 à 300 mg, le poids total des substances actives étant de 100 à 300 mg.

**16.** Forme de préparation pharmaceutique pour l'administration orale sous forme de tablettes ou de capsules selon la revendication 13, caractérisée en ce qu'elle contient 10 à 290 parties en poids du sel de calcium de l'ester monométhylique de l'acide fumarique et 290 à 10 parties en poids de fumarate de diméthyle, le poids total des substances actives étant de 100 à 300 mg.

**17.** Forme de préparation pharmaceutique pour l'administration orale sous forme de tablettes ou de capsules selon la revendication 13, caractérisée en ce qu'elle contient 10 à 250 parties en poids du sel de calcium de l'ester monométhylique de l'acide fumarique, 1 à 50 parties en poids de fumarate de diméthyle et 1 à 50 parties en poids du sel de zinc de l'ester monométhylique de l'acide fumarique, le poids total des substances actives étant de 100 à 300 mg.

**18.** Forme de préparation pharmaceutique pour l'administration orale sous forme de tablettes ou de capsules selon la revendication 13, caractérisée en ce qu'elle contient 10 à 250 parties en poids du sel de calcium de l'ester monométhylique de l'acide fumarique, 250 à 10 parties en poids de fumarate de diméthyle, 1 à 50 parties en poids du sel de magnésium de l'ester monométhylique de l'acide fumarique et 1 à 50 parties en poids du sel de zinc de l'ester monométhylique de l'acide fumarique, le poids total des substances actives étant de 100 à 300 mg.

**19.** Forme de préparation pharmaceutique pour l'administration orale selon l'une des revendications 13 à 18, caractérisée en ce qu'elle est pourvue d'un revêtement résistant au suc gastrique.

**20.** Préparations pharmaceutiques pour le traitement du psoriasis selon la revendication 13 pour l'administration orale sous forme de capsules, granulés et tablettes, pour l'administration dermique et transdermique sous forme de pommades, emplâtres, lotions et produits de douche, pour l'administration parentérale sous forme de micro-dispersions aqueuses, d'émulsions huile/eau ou de microclystères, ainsi que pour le traitement médicamenteux des cheveux et des ongles des doigts et des orteils.

**Revendications pour les Etats contractants suivants : ES, GR**

**1.** Utilisation d'un ou plusieurs du groupe des sels de calcium, de magnésium, de zinc et de fer d'ester monoalkylique d'acide fumarique répondant à la formule générale

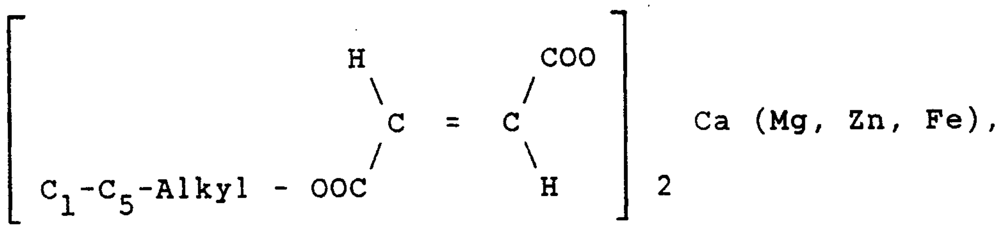

éventuellement en mélange avec du fumarate de dialkyle de formule

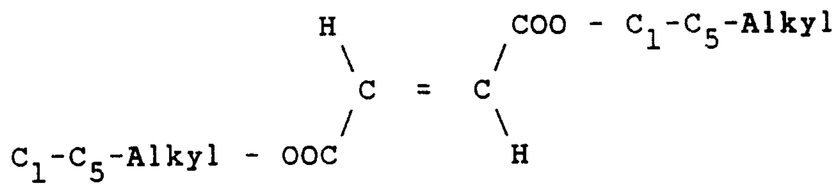

et des adjuvants et substances de support pharmaceutiques usuels pour la fabrication d'une préparation pharmaceutique pour le traitement de l'arthrite psoriasique, de la neurodermite et de l'iléite régionale de Crohn.

2. Utilisation selon la revendication 1, caractérisée en ce qu'il s'agit du sel de calcium de l'ester monoéthylique d'acide fumarique.

3. Utilisation selon la revendication 1, caractérisée en ce qu'il s'agit du sel de calcium de l'ester monométhylique d'acide fumarique.

4. Utilisation selon la revendication 1, caractérisée en ce qu'il s'agit du sel de calcium de l'ester monoéthylique d'acide fumarique en mélange avec du fumarate de diméthyle.

5. Utilisation selon la revendication 1, caractérisée en ce qu'il s'agit du sel de calcium et du sel de zinc de l'ester monométhylique d'acide fumarique en mélange avec du fumarate de diméthyle.

6. Utilisation selon la revendication 1, caractérisée en ce qu'il s'agit du sel de calcium, du sel de magnésium et du sel de zinc de l'ester monoéthylique de l'acide fumarique en mélange avec du fumarate de diméthyle.

7. Utilisation selon la revendication 1 pour la fabrication de préparations pharmaceutiques pour administration orale sous forme de tablettes ou de capsules, caractérisée en ce qu'il s'agit du sel de calcium d'ester monoalkylique d'acide fumarique en une quantité de 100 à 300 mg, le poids total des substances actives étant de 100 à 300 mg.

8. Utilisation selon la revendication 1 pour la fabrication de préparations pharmaceutiques pour administration orale sous forme de tablettes ou de capsules, caractérisée en ce qu'il s'agit de 10 à 290 parties en poids du sel de calcium d'ester monoalkylique d'acide fumarique et 290 à 10 parties en poids de fumarate de diméthyle, le poids total des substances actives étant de 100 à 300 mg.

9. Utilisation selon la revendication 1 pour la fabrication de préparations pharmaceutiques pour administration orale sous forme de tablettes ou de capsules, caractérisée en ce qu'il s'agit de 10 à 250 parties en poids du sel de calcium d'ester monoalkylique d'acide fumarique, 1 à 50 parties en poids de fumarate de diméthyle et 1 à 50 parties en poids du sel de zinc d'ester monoalkylique d'acide fumarique, le poids total des substances actives étant de 100 à 300 mg.

10. Utilisation selon la revendication 1 pour la fabrication de préparations pharmaceutiques pour administration orale sous forme de tablettes ou de capsules, caractérisée en ce qu'il s'agit de 10 à 250 parties en poids du sel de calcium d'ester monoalkylique d'acide fumarique, 250 à 10 parties en poids de fumarate de diméthyle, 1 à 50 parties en poids du sel de magnésium d'ester monoalkylique d'acide

fumarique et 1 à 50 parties en poids du sel de zinc d'ester monoalkylique d'acide fumarique, le poids total des substances actives étant de 100 à 300 mg.

11. Utilisation selon l'une des revendications précédentes pour la fabrication de préparations pharmaceutiques pour administration orale, caractérisée en ce qu'elles sont pourvues d'un revêtement résistant au suc gastrique.

12. Utilisation selon l'une des revendications précédentes pour la fabrication d'une préparation pharmaceutique pour le traitement de l'arthrite psoriasique, de la neurodermite et de l'iléite régionale de Crohn, pour administration orale sous forme de capsules, de granulés et de tablettes, pour l'administration cutanée et transcutanée sous forme de pommades, d'emplâtres, de lotions et de produits de douche, pour l'administration parentérale sous forme de microdispersions aqueuses, d'émulsions huile/eau ou d'émulsions huileuses, pour l'administration rectale sous la forme de suppositoires ou de microclystères, ainsi que pour le traitement médicamenteux des cheveux et des ongles des doigts et des orteils.

13. Procédé de fabrication d'une préparation pharmaceutique pour le traitement du psoriasis, caractérisé en ce que l'on mélange un ou plusieurs composés du groupe des sels de calcium, de magnésium, de zinc et de fer d'ester monométhylique d'acide fumarique répondant à la formule générale

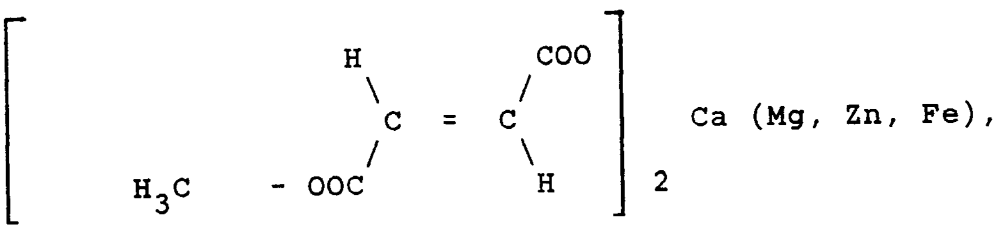

éventuellement en mélange avec du fumarate dialkyle de la formule:

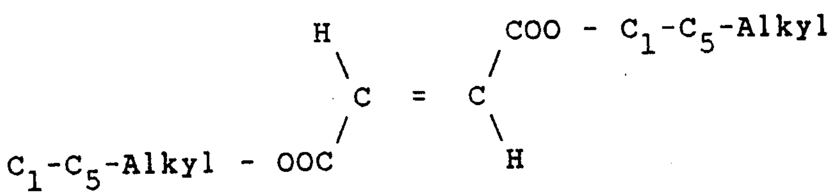

avec des substances de support, les préparations ne comprenant pas d'acide fumarique.

14. Procédé de fabrication d'une préparation pharmaceutique selon la revendication 13, caractérisé en ce que l'on utilise le sel de calcium de l'ester monoéthylique d'acide fumarique.

15. Procédé de fabrication d'une forme de préparation pharmaceutique pour administration orale sous forme de tablettes ou de capsules selon la revendication 13, caractérisée en ce qu'on utilise du sel de calcium d'ester monométhylique d'acide fumarique en une quantité de 100 à 300 mg, le poids total des substances actives étant de 100 à 300 mg.

16. Procédé de fabrication d'une forme de préparation pharmaceutique pour administration orale sous forme de tablettes ou de capsules selon la revendication 13, caractérisée en ce qu'on utilise 10 à 290 parties en poids du sel de calcium d'ester monométhylique d'acide fumarique et 290 à 10 parties en poids de fumarate de diméthyle, le poids total des substances actives étant de 100 à 300 mg.

17. Procédé de fabrication d'une forme de préparation pharmaceutique pour administration orale sous forme de tablettes ou de capsules selon la revendication 13, caractérisée en ce qu'on utilise 10 à 250 parties en poids du sel de calcium d'ester monométhylique d'acide fumarique, 1 à 50 parties en poids de fumarate de diméthyle et 1 à 50 parties en poids du sel de zinc d'ester monométhylique d'acide fumarique, le poids total des substances actives étant de 100 à 300 mg.

**18.** Procédé de fabrication d'une forme de préparation pharmaceutique pour administration orale sous forme de tablettes ou de capsules selon la revendication 13, caractérisée en ce qu'on utilise 10 à 250 parties en poids du sel de calcium d'ester monométhylique d'acide fumarique, 250 à 10 parties en poids de fumarate de diméthyle, 1 à 50 parties en poids du sel de magnésium d'ester monométhylique d'acide fumarique et 1 à 50 parties en poids du sel de zinc d'ester monométhylique d'acide fumarique, le poids total des substances actives étant de 100 à 300 mg.

**19.** Procédé de fabrication d'une forme de préparation pharmaceutique pour administration orale selon l'une des revendications 13 à 18, caractérisée en ce qu'on la munit d'un revêtement résistant au suc gastrique.

**20.** Procédé de fabrication d'une préparation pharmaceutique pour le traitement du psoriasis selon la revendication 13 pour administration orale sous forme de capsules, de granulés et de tablettes, pour l'administration cutanée et transcutanée sous forme de pommades, d'emplâtres, de lotions et de produits de douche, pour l'administration parentérale sous forme de microdispersions aqueuses, d'émulsions huile/eau ou d'émulsions huileuses, ainsi que pour le traitement médicamenteux des cheveux et des ongles des doigts et des orteils.